# EUROPEAN PATENT APPLICATION

(11) **EP 3 851 857 A1**
(43) Date of publication of application: **21.07.2021**
(21) Application number: 18933475.8
(22) Date of filing: 11.09.2018
(51) Int. Cl.: G01N 33/86

(54) **BLOOD COAGULATION ANALYSER, SAMPLE DETECTION METHOD THEREOF, AND STORAGE MEDIUM**

(71) Applicant: Shenzhen Mindray Bio-Medical Electronics Co., Ltd, Shenzhen, Guangdong 518057 (CN); Beijing Shen Mindray Medical Electronics Technology Academy Co., Ltd., Beijing 100085 (CN)
(72) Inventor: LAI, Jianxi, Shenzhen, Guangdong 518057 (CN); WU, Zhenxing, Shenzhen, Guangdong 518057 (CN); YUE, Huan, Shenzhen, Guangdong 518057 (CN); YANG, Yu, Shenzhen, Guangdong 518057 (CN); ZHAO, Jinbao, Shenzhen, Guangdong 518057 (CN); GUO, Wenheng, Shenzhen, Guangdong 518057 (CN)
(74) Representative: KIPA AB
(86) International application number: PCT/CN2018/104980
(87) International publication number: WO 2020/051761

(57) **Abstract**

A blood coagulation analyser, comprising a reaction container (100), a metal moving component (110) placed in the reaction container (100), a sample detection apparatus (200) a control apparatus (300), and a storage apparatus (400), the sample detection apparatus (200) comprising a driving coil (210) and a measuring coil (220); the driving coil (210) is configured for generating a driving electromagnetic field to drive the metal moving component (110) to oscillate in the sample to be measured in the reaction container (100); the measuring coil (220) is configured for generating an inductive electromagnetic field to detect a electrical signal produced by the oscillation of the metal moving component (110); and the control apparatus (300) is configured for acquiring the electrical signal of the metal moving component (110) in at least one continuous time period, and storing the electrical signal in the at least one continuous time period in the storage apparatus (400). Also provided are a sample detection method of the blood coagulation analyser and a storage medium.

## Description

### TECHNICAL FIELD

The present application relates to the technical field of medical devices, and in particular to a blood coagulation analyser, a sample detection method thereof, and a storage medium.

### BACKGROUND

In clinical practice, a collected sample (such as blood) can be tested for blood coagulation items by means of a blood coagulation analyser, etc., for example, the sample can be tested by means of an "optical method" or a "magnetic bead method". The principle of the test by "magnetic bead method" is as follows: a reaction vessel holding a sample is placed on a blood coagulation analyser, a reaction reagent is then added to the reaction vessel, the clotting time of the sample in the reaction vessel is measured by means of detecting the motion state of a magnetic bead in the reaction vessel, and the clotting time is provided to a user as an indicator in a report so as to implement assisting in diagnosis. However, the traditional detection methods obtain few test parameters, and once there is a problem in one of links of the sample detection, the final result will be reflected in the clotting time, so it is difficult to ensure the reliability of detection. In addition, it is difficult to assist the tester to determine the result, for example, it is not ascertained it is necessary to re-draw blood due to sample problems or to re-initiate the test due to instrument problems, etc., so the practicability needs to be improved. Therefore, the traditional detection methods are low in reliability and practicability.

### SUMMARY

On this basis, for solving the above technical problem, it is necessary to provide a blood coagulation analyser, a control method thereof, and a storage medium, so as to at least improve the practicability of the blood coagulation analyser.

A blood coagulation analyser comprises:
a reaction container, and a metal moving component placed in the reaction container, wherein the reaction container is configured to hold a mixed solution of a sample to be measured and a reaction reagent;
a sample detection apparatus, comprising a driving coil and a measuring coil, wherein the driving coil is configured to generate a driving electromagnetic field to drive the metal moving component to oscillate in the sample to be measured in the reaction container; and the measuring coil is configured to generate an inductive electromagnetic field to detect an electrical signal produced by the oscillation of the metal moving component; and
control apparatus configured for acquiring the electrical signal of the metal moving component in at least one continuous time period, and a storage apparatus used for storing the motion, and obtaining and outputting an oscillation state of the metal moving component according to the electrical signal in the at least one continuous time period,

The present application further provides a sample detection method of a blood coagulation analyser. The method comprises the following steps:
generating a driving electromagnetic field to drive a metal moving component to oscillate in a sample to be measured in a reaction container;
generating an inductive electromagnetic field to detect an electrical signal produced by the oscillation of the metal moving component in the driving electromagnetic field;
storing the electrical signal of the metal moving component in at least one continuous time period

The present application further provides computer-readable storage medium(s) with a computer program stored therein, the computer program implementing the steps in the method as any described above when being executed by a processor.

According to the blood coagulation analyser, the sample detection method thereof, and the storage medium mentioned above, the metal moving component is driven by the driving coil to oscillate in the sample to be measured in the reaction container, and the electrical signal produced by the oscillation of the metal moving component is detected by the measuring coil, such that the control apparatus can acquire the electrical signal of the metal moving component in at least one continuous time period, , the electrical signal in the at least one continuous time period is stored in the storage apparatus. The electrical signal of the metal moving component in the at least one continuous time period can reflect the detection process, so the electrical signal of the metal moving component in the at least one continuous time period can be analysed to determine whether there is a detection abnormality, thereby improving the practicability of detection.

### BRIEF DESCRIPTION OF THE DRAWINGS

FIG. 1 is a structural block diagram of a blood coagulation analyser in an embodiment;
FIG. 2 is a diagram of position relationship of a sample detection apparatus and a reaction container in an embodiment;
FIG. 3 is a schematic flowchart of a sample detection method of a blood coagulation analyser in an embodiment;
FIG. 4 is a schematic flowchart of a sample detection method of a blood coagulation analyser in another embodiment;
FIG. 5 is a schematic flowchart of a sample detection method of a blood coagulation analyser in a further embodiment;
FIG. 6 is a schematic flowchart of a method for determining a clotting time in an embodiment;
FIG. 7 is a schematic flowchart of abnormality detection method in an embodiment;
FIG. 8 is a schematic flowchart of abnormality detection method in another embodiment;
FIG. 9 is a schematic flowchart of abnormality detection method in a further embodiment;
FIG. 10 is a schematic diagram of the electrical signal of a metal moving component in a time period in an embodiment;
FIG. 11 is a schematic diagram of oscillation state of a metal moving component in an embodiment;
FIG. 12 is a schematic diagram of comparison of oscillation state of metal moving components of two samples in an embodiment;
FIG. 13 is a schematic diagram of oscillation state of a metal moving component in another embodiment;
FIG. 14 is a schematic diagram of oscillation state of a metal moving component in a further embodiment;
FIG. 15 is a schematic diagram of oscillation state of the metal moving component when a first abnormal situation exists;
FIG. 16 is a schematic diagram of oscillation state of the metal moving component when a second abnormal situation exists;
FIG. 17 is a schematic diagram of oscillation state of the metal moving component when a third abnormal situation exists;
in the figures,
100 - reaction container;
   110 - metal moving component;
200 - sample detection apparatus;
   210 - driving coil; 220 - measuring coil;
300 - control apparatus;
400 - storage apparatus;
500 - interaction apparatus.

### DESCRIPTION OF THE PREFERRED EMBODIMENTS

In order to make the objective, technical solutions, and advantages of the present application clearer, the present application will be further described below in conjunction with the accompanying drawing and the embodiments. It should be understood that the particular embodiments described here are merely intended to explain the present application but are not intended to define the present application.

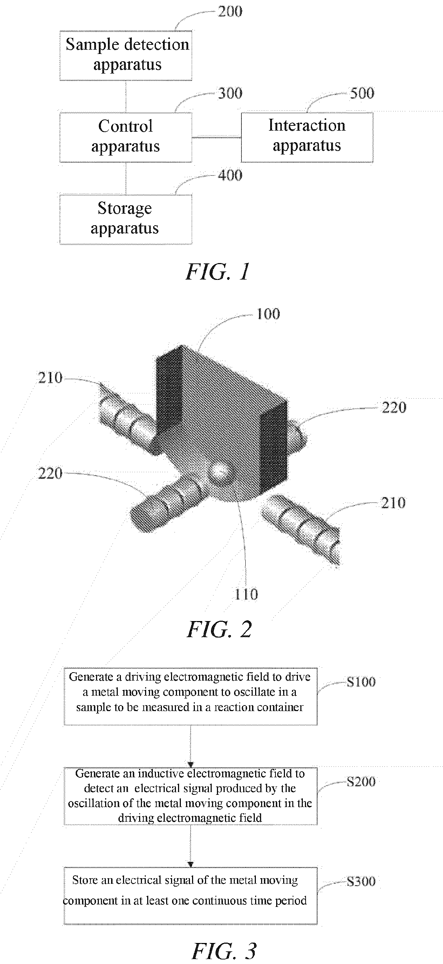

FIGS. 1 and 2 show a blood coagulation analyser according to an embodiment of the present application. The blood coagulation analyser can be configured for testing blood coagulation items so as to obtain a blood coagulation test result of a sample. For example, the blood coagulation items may include APTT (active partial thromboplastin time), PT (prothrombin time), TT (thrombin time), FIB (fibrinogen), blood coagulation factors, clinical anticoagulants or other custom items, etc., which is not specifically limited here.

The blood coagulation analyser may comprise a reaction container 100, a metal moving component 110 placed in the reaction container, a sample detection apparatus 200, a control apparatus 300 and a storage apparatus 400. The reaction container 100 is configured for holding a sample to be measured and a mixed solution thereof with a reaction reagent. For example, the reaction container 100 may be a reaction vessel, a test tube or a sample tube, etc. The metal moving component 110 is placed in the reaction container 100, and can move in the reaction container 100. The metal moving component 110 can stir the sample to be measured, the reaction reagent, etc. in the reaction container 100. In the embodiment of the present application, the metal moving component 110 may be a metal ball, such as a magnetic bead (such as a demagnetized steel bead or iron bead). Of course, in other embodiments, the metal moving component 110 may also use other shapes or materials, for example, a polyhedron, which is only illustrated here and is not specifically limited.

As shown in FIG. 2, the sample detection apparatus 200 may comprise a driving coil 210 and a measuring coil 220. The driving coil 210 is configured for generating a driving electromagnetic field to drive the metal moving component 110 to move in the sample to be measured in the reaction container 100. For example, the metal moving component 110 makes a reciprocating motion or an oscillation in the sample to be measured in the reaction container. To facilitate viewing the structure more clearly, in FIG. 2, the reaction container 100 is in a section view. Optionally, the sample detection apparatus 200 may comprise an even number of driving coil groups 210, for example, may comprise two driving coil groups 210, the two driving coil groups 210 are arranged opposite to each other at a certain distance to generate a driving electromagnetic field, and the reaction container 100 is located in the driving electromagnetic field. The driving coil 210 generates a constant alternating electromagnetic field to enable the metal moving component 110 in the reaction container 100 to oscillate. The measuring coil 220 is configured for generating an inductive electromagnetic field to detect a electrical signal produced by the oscillation of the metal moving component 110. The electrical signal refers to an electrical signal that can reflect the motion state of the metal moving component 110. Optionally, the sample detection apparatus 200 may comprise an even number of measuring coil groups 220, for example, may comprise two measuring coil groups 220, the two measuring coil groups 220 are arranged opposite to each other at a certain distance to generate an inductive electromagnetic field, and the reaction container 100 is located in the measurement electromagnetic field. The two measuring coil groups 220 include transmitting coils and receiving coils, the transmitting coils generating an excitation electromagnetic field, and the receiving coils generating a primary inductive current. When the metal moving component 110 is moving inside the reaction container 100, the receiving coils generate corresponding inductive current, and the inductive current undergoes circuit conversion and signal conditioning to obtain a voltage signal containing the motion information of the metal moving component 110, i.e., the electrical signal of the metal moving component 110. Since the metal moving component 110 moves, the characteristics of the primary inductive current is changed, and so the motion information of the metal moving component 110 can be extracted from the changed inductive current.

As shown in FIG. 2, in an embodiment, the two driving coil groups 210 and the two measuring coil groups 220 are arranged intersecting each other, the reaction container 100 is located between the two driving coil groups 210 and also located between the two measuring coil groups 220. Under the action of the driving electromagnetic field, the metal moving component 110 can move in the sample to be measured in the reaction container 100.

When the metal moving component 110 is moving in the reaction container 100, the measuring coils 220 generate corresponding inductive current, and the inductive current reflects the situation of motion of the metal moving component 110 and can be configured as the electrical signal of the metal moving component 110. Of course, it is also possible to covert the inductive current produced by the measuring coil 220 to obtain a voltage signal, and use the voltage signal as the electrical signal of the metal moving component 110.

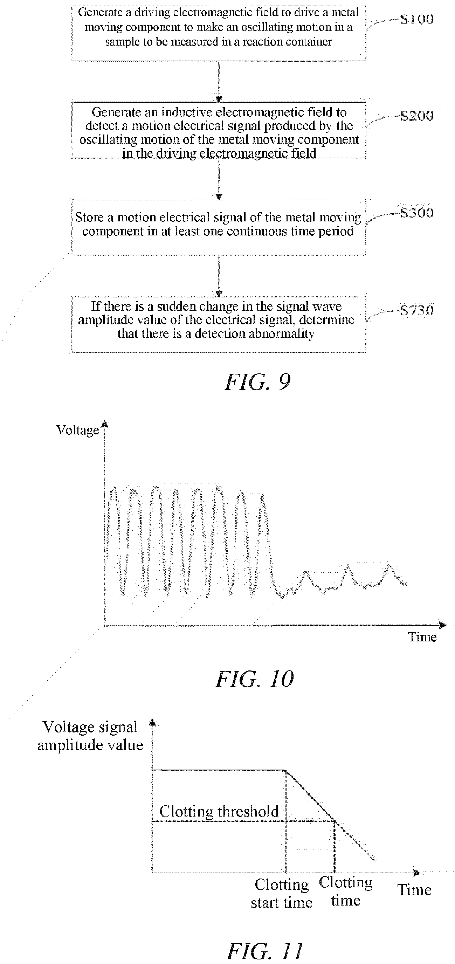

Optionally, the measuring coil 220 is connected to the control apparatus 300, and the measuring coil 220 can transfer the electrical signal of the metal moving component 110 detected thereby to the control apparatus 300. Optionally, the measuring coil 220 can be connected to the control apparatus 300 through a signal conversion circuit (not shown) and a signal conditioning circuit (not shown). For example, the signal conversion circuit can perform analog-to-digital conversion for the electrical signal of the metal moving component 110 acquired by the measuring coil 220 in at least one continuous time period, and the signal conditioning circuit can perform preprocessing, such as rectification, filtering and normalization, for the electrical signal of the metal moving component 110 acquired by the measuring coil 220 in the at least one continuous time period. FIG. 10 is a schematic diagram of the electrical signal of the metal moving component 110 in a continuous time period in an embodiment, in which the electrical signal of the metal moving component 110 may be a voltage signal.

The control apparatus 300 is configured to implement the control, such as automatic control, of the blood coagulation analyser. In the embodiment of the present application, the control apparatus 300 can acquire the electrical signal of the metal moving component 110 in at least one continuous time period, and the electrical signal in the at least one continuous time period is stored in the storage apparatus 400. In the embodiment of the present application, after a reaction reagent for a blood coagulation reaction is added to the sample to be measured (such as blood) in the reaction container 100, the blood coagulation reaction starts. Normally, as the blood coagulation reaction progresses, the production of fibrin increases, the viscosity of plasma increases, and the motion intensity of the metal moving component 110 gradually decreases. Of course, when the sample is abnormal or the instrument is abnormal, the situation of motion of the metal moving component 110 may be different accordingly. Therefore, the reaction process of the sample can be reflected by the electrical signal of the metal moving component 110 in the at least one continuous time period. By means of storing the electrical signal of the metal moving component 110 in the at least one continuous time period, the electrical signal of the metal moving component 110 in the at least one continuous time period can be analysed to determine whether there is an abnormal situation in the detection, thereby improving the reliability and practicability of detection. The at least one continuous time period mentioned here may be preset, or may be dynamically changed according to the actual situation of detection, which is not limited here.

Further, the blood coagulation analyser may also comprise a sample feed apparatus, a reaction container management apparatus, a reagent carrying apparatus, a filling apparatus, a transfer apparatus, an incubation apparatus, etc. The sample feed apparatus is configured for the automatic transfer of the sample to be measured, and the reaction container management apparatus is configured for the automatic feeding, discarding, etc. of the reaction container 100. The reagent carrying apparatus is configured for loading various reagents, such as diluents and blood coagulation reaction reagents, that are required in a blood coagulation reaction. Optionally, the reagent carrying apparatus in the embodiment of the present application may be a rotatable reagent disc, or a track transfer apparatus, etc. The filling apparatus is configured for adding reagents, etc. into the reaction container, and the transfer apparatus is configured for the transfer of the reaction container, for example, for the transfer of the reaction container from the incubation apparatus onto the sample detection apparatus. The incubation apparatus is configured for the incubation and heating of the sample to be measured and/or the reagent in the reaction container 100, for example, before the blood coagulation reaction, the reaction container holding the sample to be measured and the reaction reagent can be transferred onto the incubation apparatus for incubation to complete the incubation process under given conditions. The given conditions mentioned here may include a given temperature condition (such as 37 degrees Celsius) and a given time period (such as 5 minutes).

In an embodiment, the control apparatus 300 is further configured for obtaining oscillation state of the metal moving component 110 according to the electrical signal of the metal moving component 110 in at least one continuous time period; or the control apparatus 300 is further configured for obtaining and outputting the oscillation state of the metal moving component 110 according to the electrical signal of the metal moving component 110 in the at least one continuous time period. The oscillation state is related to the blood coagulation test result of the sample. The oscillation state of the metal moving component 110 can reflect the reaction process of the blood coagulation reaction, and the oscillation state may be a change in signal wave amplitude of the electrical signal of the metal moving component 110, etc.

Optionally, as shown in FIG. 10, the electrical signal of the metal moving component 110 may be a voltage signal. The oscillation state of the metal moving component 110 includes the change in signal wave amplitude of the electrical signal, i.e., the change in signal wave amplitude of the voltage signal, as shown in FIG. 11. In FIG. 11, the horizontal axis represents the time, and the vertical axis represents the signal wave amplitude of the electrical signal of the metal moving component 110. In the embodiment of the present application, the metal moving component 110 makes a reciprocating motion in the reaction container 100, and the electrical signal of the metal moving component 110 in a continuous time period is in a sinusoidal distribution, as shown in FIG. 10. Therefore, in a preset time interval (such as a preset motion period), the difference between the maximum value and the minimum value of the electrical signal can be taken as the motion amplitude of the metal moving component 110 in the preset time interval. As seen from FIG. 11, as the reaction of the sample to be measured in the reaction container 100 progresses, the production of fibrin increases, the viscosity of plasma increases, and the motion amplitude of the metal moving component 110 gradually decreases.

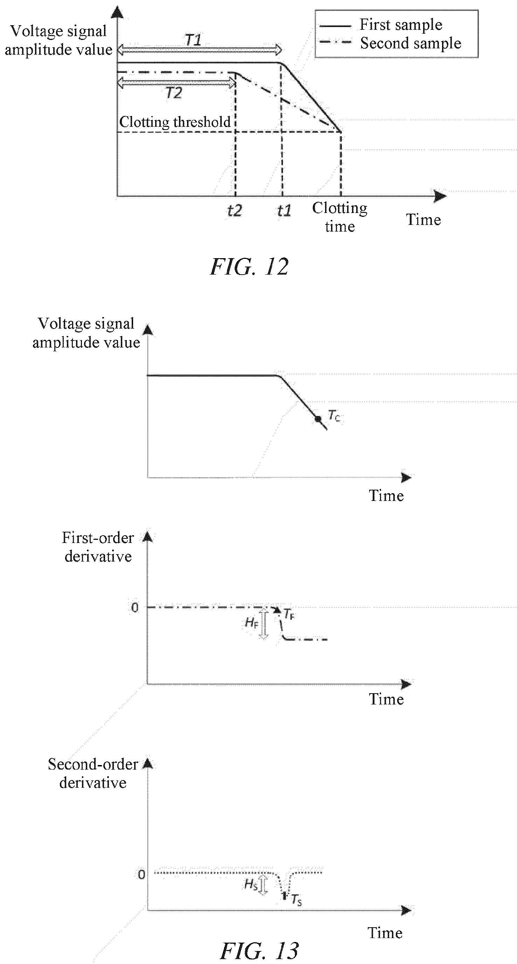

Optionally, the electrical signal of the metal moving component 110 may be a voltage signal, and the oscillation state of the metal moving component 110 includes a first-order derivative of the signal wave amplitude change of the electrical signal and/or a second-order derivative of the signal wave amplitude change of the electrical signal, as shown in FIG. 13. In the embodiment of the present application, control apparatus 300 may also obtain the maximum fibrin generation rate *H_{F}*, the time *T_{F}* corresponding to the maximum fibrin generation rate, and other information according to the first-order derivative of the signal wave amplitude change of the electrical signal of the metal moving component 110. Optionally, the time *T_{F}* corresponding to the maximum fibrin generation rate can be configured to represent a "baseline period" of the sample to be measured. In FIG. 13, the *T_{C}* represents the clotting time. In clinical practice, the "baseline period" of the sample to be measured can be configured to represent the initial fibrin generation time, and can be configured to provide clinical reference. Of course, the "baseline period" may also be the time period from the start of the blood coagulation reaction to the time when the amplitude decreases to a first threshold that can be a first threshold predetermined proportion (such as 90%) of the average amplitude value of the signal waves before the current time point. The time point when the amplitude decreases to the first threshold can be understood as the clotting start time.

Optionally, the control apparatus 300 can also obtain the maximum fibrin generation acceleration *H_{S}* and the time *T_{S}* corresponding to the maximum fibrin generation acceleration according to the second-order derivative of the signal wave amplitude change of the electrical signal of the metal moving component 110. The maximum fibrin generation acceleration can be configured to characterize the maximum activity of the factors in the common blood coagulation pathway; and the time corresponding to the maximum fibrin generation acceleration can be configured to characterize the time point corresponding to the maximum activity of the factors in the common blood coagulation pathway. In the embodiment of the present application, through the first-order derivative and the second-order derivative of the signal wave amplitude change of the electrical signal of the metal moving component 110, various process information related to the blood coagulation reaction can be obtained, which is conducive to verify the accuracy of the detection result. In some embodiments, the control apparatus 300 can also display the change in signal wave amplitude (such as the voltage signal amplitude value), and the first-order derivative and the second-order derivative of the signal wave amplitude change on a display in the form of lines, for example, corresponding curves can be displayed through three coordinate systems, as shown in FIG. 13. Of course, in other embodiments, the three curves corresponding to the change in signal wave amplitude and the first-order derivative and the second-order derivative of the signal wave amplitude change can also be displayed in the same coordinate system. In FIG. 13, in a coordinate system reflecting the signal wave amplitude change, the horizontal axis is the time, and the vertical axis is the voltage.

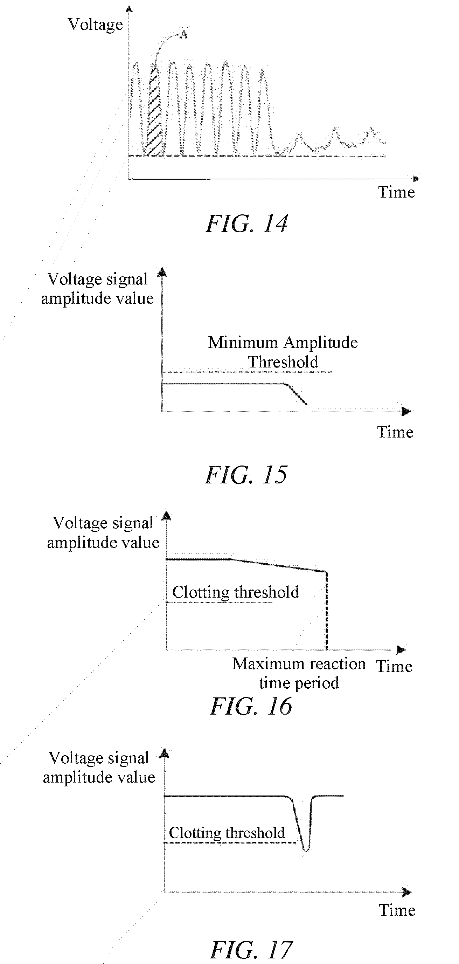

Optionally, the electrical signal of the metal moving component 110 is a voltage signal, and the oscillation state includes signal wave vibration frequency change of the electrical signal, signal phase change of the signal wave, or signal wave area change. For example, the electrical signal of the metal moving component 110 is a voltage signal, and the oscillation state of the metal moving component 110 is the signal wave area change of the electrical signal of the metal moving component 110. As shown in FIG. 14, the vertical axis in FIG. 14 represents the electrical signal (i.e., the voltage signal) of the metal moving component 110, the horizontal axis in FIG. 14 represents the time, the minimum values of the voltage signals during the sample reaction in FIG. 14 are configured to establish a baseline parallel to the horizontal axis (the broken line, parallel to the horizontal axis, in FIG. 14), a vertical line is drawn at each of the left and right lowest points of each voltage signal wave to intersect the baseline so as to form the area A of the region enclosed by the voltage signal wave and the baseline, and the area change of each signal wave is configured as the oscillation state of the metal moving component 110.

In an embodiment, the blood coagulation analyser further comprises an interaction apparatus 500 connected to the control apparatus 300. The interaction apparatus 500 is configured for displaying the oscillation state of the metal moving component 110. The interaction apparatus 500 may comprise a display, for example, a touch display panel, integrated on the blood coagulation analyser. Of course, the interaction apparatus 500 may also be a terminal, such as a desktop computer, a tablet computer or a mobile phone, connected to the control apparatus 300. Optionally, the oscillation state of the metal moving component 110 is displayed on the interaction apparatus 500 in the form of a line, and can be configured as a motion state diagram of the metal moving component 110, as shown in FIGS. 11 to 13. In this way, the user can obtain the reaction process information of the sample to be measured in the reaction container 100 through the motion state diagram of the metal moving component 110.

Optionally, the oscillation state of the metal moving component 110 is displayed on the interaction apparatus 500 in the form of a line, a chart or a table. For example, the oscillation state of the metal moving component 110 can be presented on the interaction apparatus 500 in the form of a motion curve, as shown in FIGS. 10 to 14. For another example, the oscillation state of the metal moving component 110 can be displayed on the interaction apparatus 500 in a graphic form, such as a histogram or a pie chart. For a further example, the oscillation state of the metal moving component 110 can be presented on the interaction apparatus 500 in the form of a table. Of course, in other embodiments, the oscillation state of the metal moving component 110 may also be presented in other possible forms, which are only configured for illustration here and are not configured to limit the scope of protection of the present application. Optionally, the clinical reference obtained according to the oscillation state can also be presented on the interaction apparatus. The clinical reference is the information provided to a user for clinical reference, for example, for determining the possible diseases or physical functions, for example, determining dysfibrinogenemia.

In an embodiment, the control apparatus is 300 is further configured for obtaining a blood coagulation test result of the sample according to the oscillation state. The blood coagulation test result of the sample includes the clotting time of the sample and/or the clotting start time of the sample. The clotting start time is the time interval from the start time point of the clotting reaction to the time point when the sample to be measured in the reaction container 100 starts clotting start. The clotting time is the time interval between the start time point of the clotting reaction and the end time point of the clotting reaction. For example, the start time point of the clotting reaction is the time point when a specific reaction reagent for the clotting reaction is added into the reaction container 100. The clotting start time point of the sample to be measured in the reaction container 100 can be the time point when the electrical signal of the metal moving component 110 starts to decay, and can be understood as the time point when the "baseline period" ends. For example, for the time *T_{F}* corresponding to the maximum fibrin generation rate (as shown in FIG. 13) or the time point when the electrical signal amplitude decreases to the first threshold, the first threshold may be a first threshold predetermined proportion (such as 90%) of the average amplitude value of the signal waves before the current time point. The end time point of the clotting reaction may be the time point when the amplitude decreases to a second threshold. The second threshold may be a second threshold predetermined proportion (such as 50%) of the average amplitude value of the signal waves before the current time point, and can be understood as the time point when the clotting reaction is completed.

Optionally, the control apparatus 300 can output the clotting time and the clotting start time mentioned above, which are obtained according to the oscillation state of the metal moving component 110, to the interaction apparatus 500, and the interaction apparatus 500 can present the clotting time and the clotting start time. For example, the interaction display apparatus displays a motion curve chart of the metal moving component 110 according to the oscillation state, and the clotting time and the clotting start time can be marked and presented on the motion curve chart of the metal moving component 110, as shown in FIGS. 11 to 13.

Optionally, the oscillation state of the metal moving component 110 includes the signal wave amplitude change of the electrical signal, and the control apparatus 300 can obtain the clotting time, according to current time point, when the signal wave amplitude value of the electrical signal of the metal moving component 110 at the current time point is less than or equal to the clotting threshold. Specifically, when the control apparatus 300 obtains that the signal wave amplitude value of the electrical signal of the metal moving component 110 at the current time point is less than or equal to the clotting threshold, it can be determined that the current time point is the end time point of the clotting reaction, i.e., the control apparatus 300 can determine that the clotting reaction of the sample to be measured in the reaction container 100 ends and can thus determine the above clotting time.

In an embodiment, the clotting threshold may be a preset fixed value. Then, if the signal wave amplitude value of the electrical signal of the metal moving component 110 at the current time point is less than or equal to the initial value of the clotting threshold, it can be determined that the current time point is the end time point of the clotting reaction so as to determine the above clotting time.

In an embodiment, the clotting threshold may be a variable, i.e., the clotting threshold may be dynamically updated. For example, the clotting threshold may be dynamically updated according to the signal wave amplitude value of the metal moving component 110 in each motion period. Optionally, the control apparatus 300 is further configured for obtaining the clotting threshold according to the average amplitude value of the signal waves before the signal wave at the current time point. In this way, the real-time update for the clotting threshold can improve the accuracy of the detection result. The average amplitude value may be an arithmetic average value, or may be a weighted average value or a geometric average value. In this case, the control apparatus 300 can obtain the initial value of the clotting threshold according to the average value of the signal wave amplitude values of the first N signal waves in the electrical signal of the metal moving component 110, where N is greater than or equal to 1, and N is a positive integer. Each signal wave may be an electrical signal produced by the motion of the metal moving component 110 in one oscillation period, the same below.

Specifically, the control apparatus 300 can obtain the average value of the signal wave amplitude values of the first N signal waves in the electrical signal of the metal moving component 110, and the average value may be an arithmetic average value, or may be a weighted average value or a geometric average value. Then, the control apparatus 300 may use the product of the average value of the signal wave amplitude values of the first N signal waves and the first preset proportion as the initial value of the clotting threshold. For example, the value of N is 10, the control apparatus 300 obtains the average value of the signal wave amplitude values of the first 10 signal waves of the electrical signal of the metal moving component 110, and uses the product of the average value of the signal wave amplitude values of the first 10 signal waves and the first preset proportion (such as 50%) as the initial value of the clotting threshold.

For example, after the initial value of the clotting threshold is determined and when a new signal wave (the (N + 1)th) is detected, the control apparatus 300 can obtain the signal wave amplitude value at the current time point, and uses the product of the signal wave amplitude value at the current time point and the second preset proportion (such as 50%) as a regulating variable of the clotting threshold. The second preset proportion may be the same as or different from the first preset proportion. Then, the control apparatus 300 may perform a weighted average calculation on the initial value of the clotting threshold and the regulating variable of the clotting threshold to obtain an updated clotting threshold so as to implement the dynamic update of the clotting threshold and improve the accuracy of the detection result.

In an embodiment, the control apparatus 300 is further configured for outputting the clinical reference according to the oscillation state. The clinical reference can be configured to assist a user in the diagnosis of diseases. For example, the control apparatus 300 can obtain the "baseline period" corresponding to the sample to be measured according to the oscillation state, compare the "baseline period" of the sample to be measured with a preset reference value to perform screening of an abnormal disease, and output the screening result of the abnormal disease as clinical reference to assist a user in the diagnosis of diseases. As mentioned above, the "baseline period" of the sample to be measured can refer to the time period in which, after the blood coagulation reaction starts, the amplitude value of the electrical signal of the metal moving component decreases to the first threshold predetermined proportion (such as 90%) of the average amplitude value of the signal waves before the current time point.

FIG. 12 is a schematic diagram of comparison of oscillation state of metal moving components of two samples in an embodiment. T1 represents the "baseline period" corresponding to the first sample, t1 represents the clotting start time point of the first sample, T2 represents the "baseline period" of the second sample, and t2 represents the clotting start time point of the second sample. As seen from FIG. 12, although the first sample and the second sample have equal clotting time, the second sample has a faster initial fibrin generation speed, but has a slower subsequent replenishment speed. In the embodiment of the present application, by means of determining and/or outputting the oscillation state of the metal moving component 110, the other information, such as the clotting start time, other than the clotting time can be obtained through the oscillation state, and can be configured to assist the clinical diagnosis to improve the practicability. Moreover, through the first-order derivative of the signal wave amplitude change of the electrical signal and/or the second-order derivative of the signal wave amplitude change of the electrical signal, various process information related to the blood coagulation reaction can be obtained, for example, as shown in FIG. 13, the maximum fibrin generation rate *H_{F}* and the time *T_{F}* corresponding to the maximum fibrin generation rate, and the maximum fibrin generation acceleration *H_{S}* and the time *T_{S}* corresponding to the maximum fibrin generation acceleration mentioned above, which can effectively improve the practicability of detection and has a positive effect in clinical practice.

In an embodiment, the control apparatus 300 is further configured for determining whether there is a detection abnormality according to the oscillation state of the metal moving component 110. Further, the control apparatus 300 is further configured for, when it is determined that there is a detection abnormality according to the oscillation state, outputting abnormal information and/or outputting operating recommendation and/or giving an alarm. For example, the abnormal information may be the occurrence of detection abnormality, such as "bubbles in the sample to be measured". The operating recommendation may be "please check the sample to be measured and re-test", etc. Still further, the abnormal information and the operating recommendation may be displayed through the interaction apparatus 500. Optionally, the control apparatus 300 may also control an alarm light source and/or sound source provided on the blood coagulation analyser to give an alarm when there is a detection abnormality so as to inform the user of the detection abnormality.

Optionally, the oscillation state of the metal moving component 110 includes a change in signal wave amplitude of the electrical signal, and the control apparatus 300 is further configured for, when all the signal wave amplitude values of the first M signal waves of the electrical signal are less than the preset minimum amplitude threshold, determining that there is a detection abnormality; wherein M is greater than or equal to 1, and M is a positive integer, for example, M may be 20. The value of M depends on the user's determination for the motion oscillation start period, and the abnormality is determined by means of determining whether the oscillation start amplitude in the motion oscillation start period is lower than the minimum amplitude threshold. As shown in FIG. 15, after the blood coagulation reaction starts, if the oscillation start amplitude value of the electrical signal is less than the minimum amplitude threshold, the control apparatus 300 determines that there is a detection abnormality. When the control apparatus 300 determines that all the signal wave amplitude values of the first M signal waves of the electrical signal of the metal moving component 110 are less than the preset minimum amplitude threshold, there may be bubbles in the sample to be measured in the reaction container 100, which causes the motion amplitude of the metal moving component 110 to be lower than the normal value. Then the control apparatus 300 can determine that the oscillation start amplitude of the metal moving component 110 is too small, so there is a detection abnormality. When the control apparatus 300 determines that there is a detection abnormality, the control apparatus outputs abnormal information to the interaction apparatus 500 and gives an alarm. The user can examine the sample to be measured for processing, and then perform an operation through the interaction apparatus 500 to re-initiate the test.

Optionally, the oscillation state includes a change in signal wave amplitude of the electrical signal, and the control apparatus 300 is further configured for determining that there is a detection abnormality when the signal wave amplitude value of the electrical signal starts to decay in the preset maximum reaction time period and when the signal wave amplitude value of the electrical signal of the metal moving component 110 in the preset maximum reaction time period remains greater than the clotting threshold, as shown in FIG. 16. When the signal wave amplitude value of the electrical signal of the metal moving component 110 starts to decay in the preset maximum reaction time period (for example, the preset maximum reaction time period may be 200 seconds), and when the signal wave amplitude value of the electrical signal of the metal moving component 110 in the preset maximum reaction time period remains greater than the clotting threshold, the clotting reaction may have a slow process due to the improper collection of the sample to be measured (for example, the sample to be measured for the TT item is collected from a heparin indwelling needle) or the excessive low content of blood coagulation factors/fibrin in the sample to be measured. Then, the control apparatus 300 can determine that there is a detection abnormality according to the oscillation state of the metal moving component 110. When it is determined that there is a detection abnormality, the control apparatus 300 outputs the abnormal information to the interaction apparatus 500, and gives an alarm to prompt the user to initiate a re-test and/or automatically initiate a new test for detection and analysis of the sample to be measured again.

For example, if it is determined that the abnormality is caused by improper collection of the sample to be measured (for example, the sample to be measured for the TT item is collected from a heparin indwelling needle), then it is necessary to re-draw a qualified sample for testing. The control apparatus 300 can output the abnormal information to the interaction apparatus 500 and give an alarm. The user can re-initiate the test through the interaction apparatus 500 after replacing the sample. If it is determined that the detection abnormality is caused by the excessive low content of the blood coagulation factors or fibrin in the sample to be measured, the control apparatus 300 can automatically initiate a new test for detection and analysis of the sample to be measured again. For example, when the content of the blood coagulation factors or fibrin in the sample to be measured is excessive low, and the initiated detection item (such as FIB items) allows "concentration", then the control apparatus 300 can automatically initiate a "concentration re-test". It should be clear that the "concentration" mentioned here refers to keeping the total volume of the sample to be measured and the diluent while reducing the absolute volume of the diluent, thereby increasing the content of the blood coagulation factors or fibrin in the sample to be measured.

Optionally, the oscillation state of the metal moving component 110 includes a change in signal wave amplitude of the electrical signal, and the control apparatus 300 is further configured for, when there is a sudden change in the signal wave amplitude value of the electrical signal, determining that there is a detection abnormality. The case where there is a sudden change in the signal wave amplitude value of the electrical signal of the metal moving component 110 refers to the case where the amplitude of the metal moving component 110 decreases to the clotting threshold or below at first and then increases to the average amplitude value of the signal waves before the change, as shown in FIG. 17.

Specifically, when the control apparatus 300 determines that there is a sudden change in the signal wave amplitude value of the electrical signal of the metal moving component 110, the detection abnormality may be caused by the clotting time of the sample to be measured exceeding the "linear range" of the blood coagulation analyser. Some test items (such as FIB items) of the instrument have the concept of "linear range". In the "linear range", the clotting time has a linear relationship with the concentration of fibrinogen in the sample. At this time, the metal moving component has a normal motion rule, that is, the amount of fibrin generated is sufficient, the binding force on the metal moving component can be offset with the magnetic field driving force exerted by the driving coil, and the metal moving component remains stationary after the blood coagulation reaction is completed. However, outside the "linear range", for example, the sample has a low fibrinogen content, and when approaching the end point of clotting, the amount of fibrin generated is low, the binding force on the metal moving component is cannot balance the magnetic field driving force exerted by the driving coil, and at this time, the fibrin will be "pulled off' by the motion of the metal moving component, and the metal moving component after breaking through the fibrin binding will gradually resume the constant-amplitude oscillation. Then, after it is determined that there is a detection abnormality, the control apparatus 300 outputs the abnormal information to the interaction apparatus 500 and gives an alarm, and can prompt the user to initiate a re-test or automatically initiate a new test for detection and analysis of the sample to be measured again.

In an embodiment, the control apparatus 300 is further configured for outputting the activity information and/or concentration information of the sample to be measured according to the oscillation state. Optionally, the activity information and/or concentration information of the sample to be measured may also be displayed through an interaction apparatus 500.

Corresponding to the above blood coagulation analyser, the embodiment of the present application further provides a sample detection method of a blood coagulation analyser. The method can be applied to the above blood coagulation analyser to implement the test of blood coagulation items so as to obtain a blood coagulation test result of the sample. Specifically, as shown in FIG. 3, the above method may comprise the following steps.

S100, a driving electromagnetic field is generated to drive a metal moving component to oscillate in a sample to be measured in a reaction container.

Specifically, the driving electromagnetic field can be generated by an even number of driving coil groups 210 arranged opposite to each other. For example, the driving electromagnetic field is generated by two driving coil groups 210 arranged opposite to each other, and the driving electromagnetic field can drive the metal moving component 110 to move in the sample to be measured in the reaction container. For example, the metal moving component 110 makes a reciprocating motion or an oscillation in the sample to be measured in the reaction container. Optionally, when a blood coagulation detection is required, the reaction container holding the sample to be measured can be transferred to the sample detection apparatus 200, then a reagent for the blood coagulation reaction is added into the reaction container 100, and then the blood coagulation detection starts. Normally, after the blood coagulation reaction starts, the driving electromagnetic field can drive the metal moving component 110 to move in the sample to be measured in the reaction container, and as the blood coagulation reaction progresses, since the fibrin generated will hinder the motion of the metal moving component, the amplitude of motion of the metal moving component will gradually decrease.

S200, an inductive electromagnetic field is generated to detect the electrical signal produced by the oscillation of the metal moving component in the driving electromagnetic field.

Specifically, the inductive electromagnetic field can be generated by an even number of measuring coil groups 220 arranged opposite to each other, for example, generated by two measuring coil groups 220 arranged opposite to each other. The inductive electromagnetic field can be configured for detecting the electrical signal produced by the oscillation of the metal moving component 110 in the driving electromagnetic field. The electrical signal refers to an electrical signal that can reflect the motion state of the metal moving component 110. Optionally, the measuring coil 210 is connected to the control apparatus 300, and the measuring coil 220 can transfer the electrical signal of the metal moving component detected thereby to the control apparatus 300. Optionally, the measuring coil 220 can be connected to the control apparatus 300 through a signal conversion circuit and a signal conditioning circuit. For example, the signal conversion circuit can perform analog-to-digital conversion for the electrical signal of the metal moving component acquired by the measuring coil in at least one continuous time period, and the signal conditioning circuit can perform preprocessing, such as rectification, filtering and normalization, for the electrical signal of the metal moving component acquired by the measuring coil in the at least one continuous time period. FIG. 10 is a schematic diagram of the electrical signal of the metal moving component in a continuous time period in an embodiment.

S300, the electrical signal of the metal moving component in at least one continuous time period is stored.

Specifically, the electrical signal of the metal moving component in the at least one continuous time period can be stored in the storage apparatus 400. Normally, after a specific reaction reagent is added to the sample to be measured (such as blood) in the reaction container, the blood coagulation reaction starts, and as the blood coagulation reaction progresses, the production of fibrin increases, the viscosity of plasma increases, and the motion intensity of the metal moving component 110 gradually decreases. Of course, when the sample is abnormal or the instrument is abnormal, the situation of motion of the metal moving component 110 may be different accordingly. Therefore, the electrical signal of the metal moving component 110 in the at least one continuous time period can reflect the reaction process of the sample. By means of storing the electrical signal of the metal moving component in the at least one continuous time period, the electrical signal of the metal moving component in the at least one continuous time period can be analysed to determine whether there is an abnormal situation in the detection, thereby improving the reliability and practicability of detection. The at least one continuous time period mentioned here may be preset, or may be dynamically changed according to the actual situation of detection, which is not limited here.

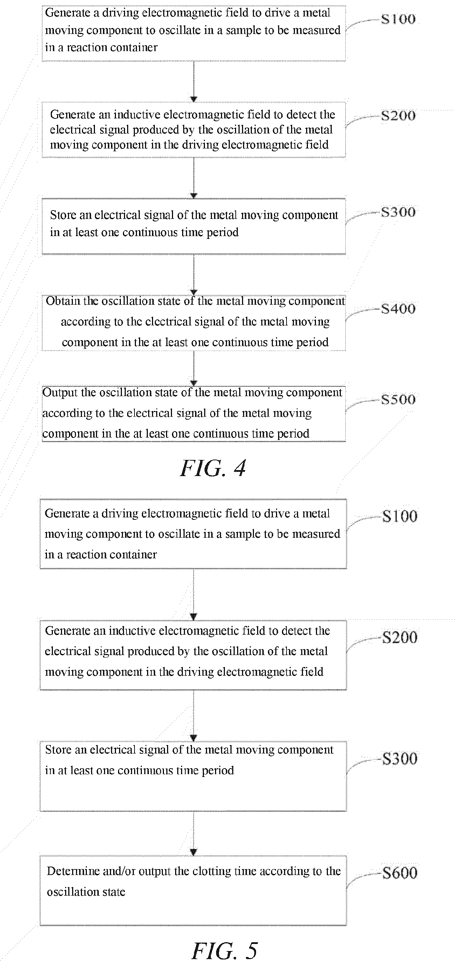

In an embodiment, as shown in FIG. 4, the above method further comprises the following steps.

S400, the oscillation state of the metal moving component 110 is obtained according to the electrical signal of the metal moving component 110 in the at least one continuous time period. The oscillation state is related to the blood coagulation test result of the sample. Optionally, as shown in FIG. 10, the electrical signal of the metal moving component 110 may be a voltage signal. The oscillation state of the metal moving component 110 includes a change in signal wave amplitude of the electrical signal, i.e., the change in signal wave amplitude of the voltage signal, as shown in FIG. 11. Of course, the oscillation state of the metal moving component 110 may also be other characteristic parameters that can reflect the motion track of the metal moving component 110.

500, when the oscillation state of the metal moving component 110 is obtained according to the electrical signal of the metal moving component 110 in the at least one continuous time period, the oscillation state of the metal moving component 110 may also be output. For example, the control apparatus 300 can output the oscillation state of the metal moving component 110 to the interaction apparatus for displaying. For another example, the control apparatus 300 can also output the oscillation state of the metal moving component 110 to a printer or other apparatuses.

In an embodiment, the oscillation state of the metal moving component 110 may also be output in the form of a line, as shown in FIGS. 11 to 13. In other embodiments, the oscillation state of the metal moving component 110 may also be output in the form of a chart or a table. For example, the oscillation state of the metal moving component 110 can be displayed on the interaction apparatus 500 in a graphic form, such as a histogram or a pie chart. For another example, the oscillation state of the metal moving component 110 can be presented on the interaction apparatus 500 in the form of a table. Of course, the oscillation state of the metal moving component 110 may also be presented in other possible forms, which are only configured for illustration here and are not configured to limit the scope of protection of the present application.

Optionally, as shown in FIG. 10, the electrical signal of the metal moving component 110 may be a voltage signal. The oscillation state of the metal moving component 110 includes a change in signal wave amplitude of the electrical signal, i.e., the change in signal wave amplitude of the voltage signal, as shown in FIG. 11. In FIG. 11, the horizontal axis represents the time, and the vertical axis represents the signal wave amplitude of the electrical signal of the metal moving component 110. In the embodiment of the present application, the metal moving component 110 makes a reciprocating motion in the reaction container 100, and the electrical signal of the metal moving component 110 in a continuous time period is in a sinusoidal distribution, as shown in FIG. 10. Therefore, in a preset time interval (such as a preset motion period), the difference between the maximum value and the minimum value of the electrical signal can be taken as the motion amplitude of the metal moving component 110 in the preset time interval. As seen from FIG. 11, as the reaction of the sample to be measured in the reaction container 100 progresses, the production of fibrin increases, the viscosity of plasma increases, and the motion amplitude of the metal moving component 110 gradually decreases.

Optionally, the electrical signal of the metal moving component 110 may be a voltage signal, and the oscillation state of the metal moving component 110 includes a first-order derivative of the signal wave amplitude change of the electrical signal and/or a second-order derivative of the signal wave amplitude change of the electrical signal, as shown in FIG. 13. In the embodiment of the present application, control apparatus 300 may also obtain the maximum fibrin generation rate *H_{F}*, the time *T_{F}* corresponding to the maximum fibrin generation rate, and other information according to the first-order derivative of the signal wave amplitude change of the electrical signal of the metal moving component 110. Optionally, the time *T_{F}* corresponding to the maximum fibrin generation rate can be configured to represent a "baseline period" of the sample to be measured. In FIG. 13, the *T_{C}* represents the clotting time. In clinical practice, the "baseline period" of the sample to be measured can be configured to represent the initial fibrin generation time, and can be configured to provide clinical reference. Of course, the "baseline period" may also be the time period from the start of the blood coagulation reaction to the time when the amplitude decreases to a first threshold that can be a first threshold predetermined proportion (such as 90%) of the average amplitude value of the signal waves before the current time point. The time point when the amplitude decreases to the first threshold can be understood as the clotting start time.

Optionally, the control apparatus 300 can also obtain the maximum fibrin generation acceleration *H_{S}* and the time *T_{S}* corresponding to the maximum fibrin generation acceleration according to the second-order derivative of the signal wave amplitude change of the electrical signal of the metal moving component 110. The maximum fibrin generation acceleration can be configured to characterize the maximum activity of the factors in the common blood coagulation pathway, and the time corresponding to the maximum fibrin generation acceleration can be configured to characterize the time point corresponding to the factors in the common blood coagulation pathway having the maximum activity. In the embodiment of the present application, through the first-order derivative and the second-order derivative of the signal wave amplitude change of the electrical signal of the metal moving component 110, various process information related to the blood coagulation reaction can be obtained, which is conducive to assist the clinical diagnosis and improve the practicability.

Optionally, the electrical signal of the metal moving component 110 is a voltage signal, and the oscillation state includes signal wave vibration frequency change of the electrical signal, signal phase change information of the signal wave, or signal wave area change information. For example, the electrical signal of the metal moving component 110 is a voltage signal, and the oscillation state of the metal moving component 110 is the signal wave area change of the electrical signal of the metal moving component 110. As shown in FIG. 14, the vertical axis in FIG. 14 represents the electrical signal (i.e., the voltage signal) of the metal moving component 110, the horizontal axis in FIG. 14 represents the time, the minimum values of the voltage signals during the sample reaction in FIG. 14 are configured to establish a baseline parallel to the horizontal axis (the broken line, parallel to the horizontal axis, in FIG. 14), a vertical line is drawn at each of the left and right lowest points of each voltage signal wave to intersect the baseline so as to form the area A of the region enclosed by the voltage signal and the baseline in the calculation unit period, and the area of each signal wave is configured as the oscillation state of the metal moving component 110.

In an embodiment, as shown in FIG. 5, the above method comprises the following step.

S600, the blood coagulation test result of the sample is obtained according to the oscillation state, the blood coagulation test result of the sample including the clotting time of the sample. The clotting time is the time interval between the start time point of the clotting reaction and the end time point of the clotting reaction. For example, the start time point of the clotting reaction is the time point when the specific reaction reagent is added to the clotting reaction in the reaction container. The end time point of the clotting reaction may be the time point when the amplitude decreases to a second threshold. The second threshold may be a second threshold predetermined proportion (such as 50%) of the average amplitude value of the signal waves before the current time point, and can be understood as the time point when the clotting reaction is completed.

Further, the above method further comprises the following step:
obtaining the blood coagulation test result of the sample according to the oscillation state, the blood coagulation test result of the sample including the clotting start time of the sample. The clotting start time is the time interval from the start time point of the clotting reaction to the time point when the sample to be measured in the reaction container starts clotting start. For example, the start time point of the clotting reaction is the time point when the specific reaction reagent is added to the clotting reaction in the reaction container. The clotting start time point of the sample to be measured in the reaction container can be the time point when the electrical signal of the metal moving component 110 starts to decay, and can be understood as the time point when the "baseline period" ends. For example, for the time *T_{F}* corresponding to the maximum fibrin generation rate (as shown in FIG. 13) or the time point when the electrical signal amplitude decreases to the first threshold, the first threshold may be a first threshold predetermined proportion (such as 90%) of the average amplitude value of the signal waves before the current time point.

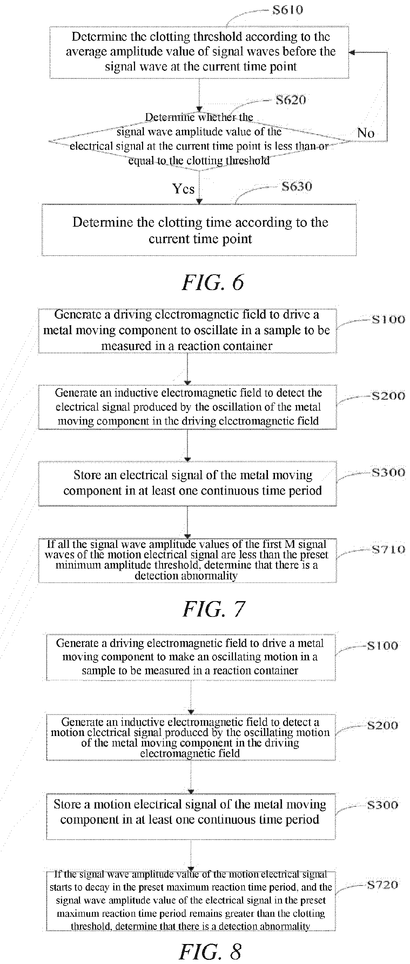

In an embodiment, the clotting threshold is a variable, i.e., the clotting threshold can be dynamically updated. For example, the clotting threshold may be dynamically updated according to the signal wave amplitude value of the metal moving component 110 in each motion period. In this way, the real-time update for the clotting threshold can improve the accuracy of the detection result. Optionally, the oscillation state of the metal moving component 110 includes the change in signal wave amplitude of the electrical signal, and as shown in FIG. 6, the above method further comprises the following steps.

S610, the clotting threshold is obtained according to the average amplitude value of signal waves before the signal wave at the current time point. The average amplitude value may be an arithmetic average value, or may be a weighted average value or a geometric average value.

S620, whether the signal wave amplitude value of the electrical signal of the metal moving component 110 at the current time point is less than or equal to the clotting threshold is determined.

If the signal wave amplitude value of the electrical signal at the current time point is less than or equal to the clotting threshold, step S630 is performed to determine the clotting time according to the current time point. Specifically, when it is determined that the signal wave amplitude value of the electrical signal of the metal moving component 110 at the current time point is less than or equal to the clotting threshold, it is determined that the current time point is the end time point of the clotting reaction, that is, it can be determined that the clotting reaction of the sample to be measured in the reaction container ends, and thus the above clotting time can be determined.

In the embodiment of the present application, when the signal wave amplitude value of the electrical signal of the metal moving component 110 at the current time point is greater than the clotting threshold, the process can return to step S610, and then the above steps S610 to S620 are repeated until the signal wave amplitude value of the electrical signal of the metal moving component 110 at the current time point is less than or equal to the clotting threshold, and then the clotting time can be determined according to the current time point.

Optionally, the control apparatus can obtain the initial value of the clotting threshold according to the average value of the signal wave amplitude values of the first N signal waves in the electrical signal of the metal moving component 110, where N is greater than or equal to 1, and N is a positive integer. Specifically, the control apparatus can determine the average value of the signal wave amplitude values of the first N signal waves in the electrical signal of the metal moving component 110, and the average value may be an arithmetic average value, or may be a geometric average value. Then, the control apparatus may use the product of the average value of the signal wave amplitude values of the first N signal waves and the first preset proportion as the initial value of the clotting threshold. For example, the value of N is 10, the control apparatus determines the average value of the signal wave amplitude values of the first 10 signal waves of the electrical signal of the metal moving component 110, and uses the product of the average value of the signal wave amplitude values of the first 10 signal waves and the first preset proportion (such as 50%) as the initial value of the clotting threshold.

For example, after the initial value of the clotting threshold is determined and when a new signal wave (the (N + 1)th) is detected, the control apparatus 300 can obtain the signal wave amplitude value at the current time point, and uses the product of the signal wave amplitude value at the current time point and the second preset proportion (such as 50%) as a regulating variable of the clotting threshold. The second preset proportion may be the same as or different from the first preset proportion. Then, the control apparatus 300 may perform a weighted average calculation on the initial value of the clotting threshold and the regulating variable of the clotting threshold to obtain an updated clotting threshold so as to implement the dynamic update of the clotting threshold and improve the accuracy of the detection result.

In an embodiment, the clotting threshold is a preset fixed value, and for example, the clotting threshold is the above initial value of the clotting threshold. Then, if the signal wave amplitude value of the electrical signal of the metal moving component 110 at the current time point is less than or equal to the initial value of the clotting threshold, it can be determined that the current time point is the end time point of the clotting reaction so as to determine the above clotting time.

In an embodiment, the control apparatus 300 is further configured for outputting the clinical reference according to the oscillation state. The clinical reference is configured to assist a user in the diagnosis of diseases. For example, the control apparatus can obtain the "baseline period" corresponding to the sample to be measured according to the oscillation state, compare the "baseline period" of the sample to be measured with a preset reference value to perform screening of an abnormal disease, and output the screening result of the abnormal disease as clinical reference to assist a user in the diagnosis of diseases. As mentioned above, the "baseline period" of the sample to be measured can refer to the time period in which, after the blood coagulation reaction starts, the amplitude value of the electrical signal of the metal moving component 110 decreases to the first threshold predetermined proportion (such as 90%) of the average amplitude value of the signal waves before the current time point.

FIG. 12 is a schematic diagram of comparison of oscillation state of metal moving components 110 of two samples in an embodiment. T1 represents the "baseline period" corresponding to the first sample, t1 represents the clotting start time point of the first sample, T2 represents the "baseline period" of the second sample, and t2 represents the clotting start time point of the second sample. As seen from FIG. 12, although the first sample and the second sample have equal clotting time, the second sample has a faster initial fibrin generation speed, but has a slower subsequent replenishment speed. In the embodiment of the present application, by means of outputting the oscillation state of the metal moving component 110, the other information, such as the clotting start time, other than the clotting time can be obtained through the oscillation state, and can be configured to assist the clinical diagnosis to improve the practicability. Moreover, through the first-order derivative of the signal wave amplitude change of the electrical signal and/or the second-order derivative of the signal wave amplitude change of the electrical signal, various process information related to the blood coagulation reaction can be obtained, for example, as shown in FIG. 13, the maximum fibrin generation rate *H_{F}* and the time *T_{F}* corresponding to the maximum fibrin generation rate, and the maximum fibrin generation acceleration *H_{S}* and the time *T_{S}* corresponding to the maximum fibrin generation acceleration mentioned above, which can effectively improve the practicability of detection and has a positive effect in clinical practice.

In an embodiment, as shown in FIGS. 7 to 9, the above method comprises the following step:
determining whether there is a detection abnormality according to the oscillation state.

Further, the above method further comprises the following step: outputting abnormal information and/or outputting operating recommendation and/or giving an alarm according to the oscillation state. For example, the abnormal information may be the occurrence of detection abnormality, such as "bubbles in the sample to be measured". The operating recommendation may be "please check the sample to be measured and re-test", etc. Still further, the abnormal information and the operating recommendation may be displayed through the interaction apparatus. Optionally, the control apparatus 300 may also control an alarm light source and/or sound source provided on the blood coagulation analyser to give an alarm when there is a detection abnormality so as to inform the user of the detection abnormality.

Optionally, the oscillation state includes a change in signal wave amplitude of the electrical signal, and as shown in FIG. 7, the above method further comprises the following step.

S710, if all the signal wave amplitude values of the first M signal waves of the electrical signal are less than the preset minimum amplitude threshold, it is determined that there is a detection abnormality; wherein M is greater than or equal to 1, and M is a positive integer. For example, M may be 20. The value of M depends on the user's determination for the motion oscillation start period, and the abnormality is determined by means of determining whether the oscillation start amplitude in the motion oscillation start period is lower than the minimum amplitude threshold.

As shown in FIG. 15, after the blood coagulation reaction starts, if the oscillation start amplitude value of the electrical signal is less than the minimum amplitude threshold, the control apparatus 300 determines that there is a detection abnormality. When the control apparatus 300 determines that all the signal wave amplitude values of the first M signal waves of the electrical signal of the metal moving component 110 are less than the preset minimum amplitude threshold, there may be bubbles in the sample to be measured of the reaction container, which causes the motion amplitude of the metal moving component 110 is lower than the normal value. Then the control apparatus 300 can determine that the oscillation start amplitude of the metal moving component 110 is too small, so there is a detection abnormality. When it is determined that there is a detection abnormality, the control apparatus 300 outputs the abnormal information to the interaction apparatus and gives an alarm. The user can examine the sample to be measured for processing, and then perform an operation through the interaction apparatus to re-initiate the test.

Optionally, the oscillation state of the metal moving component 110 includes a change in signal wave amplitude of the electrical signal, and as shown in FIG. 8, the above method further comprises the following step:
S720, when the signal wave amplitude value of the electrical signal starts to decay in the preset maximum reaction time period, and the signal wave amplitude value of the electrical signal in the preset maximum reaction time period remains greater than the clotting threshold, it is determined that there is a detection abnormality.

As shown in FIG. 16, when the signal wave amplitude value of the electrical signal of the metal moving component 110 starts to decay in the preset maximum reaction time period (for example, the preset maximum reaction time period may be 200 seconds), and when the signal wave amplitude value of the electrical signal of the metal moving component 110 in the preset maximum reaction time period remains greater than the clotting threshold, the clotting reaction may have a slow process due to the improper collection of the sample to be measured (for example, the sample to be measured for the TT item is collected from a heparin indwelling needle) or the excessive low content of blood coagulation factors/fibrin in the sample to be measured. Then, the control apparatus 300 can determine that there is a detection abnormality according to the oscillation state of the metal moving component 110. When it is determined that there is a detection abnormality, the control apparatus 300 outputs the abnormal information to the interaction apparatus and gives an alarm to prompt the user to initiate a re-test and/or automatically initiate a new test for detection and analysis of the sample to be measured again.

For example, if it is determined that the abnormality is caused by improper collection of the sample to be measured (for example, the sample to be measured for the TT item is collected from a heparin indwelling needle), then it is necessary to re-draw a qualified sample for testing. The control apparatus 300 can output the abnormal information to the interaction apparatus and give an alarm. The user can re-initiate the test through the interaction apparatus after replacing the sample. If it is determined that the detection abnormality is caused by the excessive low content of the blood coagulation factors or fibrin in the sample to be measured, the control apparatus 300 can automatically initiate a new test for detection and analysis of the sample to be measured again. For example, when the content of the blood coagulation factors or fibrin in the sample to be measured is excessive low, and the initiated detection item (such as FIB items) allows "concentration", then the control apparatus 300 can automatically initiate a "concentration re-test". It should be clear that the "concentration" mentioned here refers to keeping the total volume of the sample to be measured and the diluent while reducing the absolute volume of the diluent, thereby increasing the content of the blood coagulation factors or fibrin in the sample to be measured.

Optionally, the oscillation state of the metal moving component 110 includes a change in signal wave amplitude of the electrical signal, and as shown in FIG. 9, the above method further comprises the following step.

S730, if there is a sudden change in the signal wave amplitude value of the electrical signal, it is determined that there is a detection abnormality. The case where there is a sudden change in the signal wave amplitude value of the electrical signal of the metal moving component 110 refers to the case where the amplitude of the metal moving component 110 decreases to the clotting threshold or below at first and then increases to the average amplitude value of the signal waves before the change, as shown in FIG. 17.

Specifically, when the control apparatus 300 determines that there is a sudden change in the signal wave amplitude value of the electrical signal of the metal moving component 110, the detection abnormality may be caused by the clotting time of the sample to be measured exceeding the "linear range" of the blood coagulation analyser. Some test items (such as FIB items) of the instrument have the concept of "linear range". In the "linear range", the clotting time has a linear relationship with the concentration of fibrinogen in the sample. At this time, the metal moving component has a normal motion rule, that is, the amount of fibrin generated is sufficient, the binding force on the metal moving component can be offset with the magnetic field driving force exerted by the driving coil, and the metal moving component remains stationary after the blood coagulation reaction is completed. However, outside the "linear range", for example, the sample has a low fibrinogen content, and when approaching the end point of clotting, the amount of fibrin generated is low, the binding force on the metal moving component is cannot balance the magnetic field driving force exerted by the driving coil, and at this time, the fibrin will be "pulled off' by the motion of the metal moving component, and the metal moving component after breaking through the fibrin binding will gradually resume the constant-amplitude oscillation. Then, after it is determined that there is a detection abnormality, the control apparatus 300 outputs the abnormal information to the interaction apparatus and gives an alarm, and can prompt the user to initiate a re-test or automatically initiate a new test for detection and analysis of the sample to be measured again.

In an embodiment, the above-mentioned method further comprises the following step: outputting activity information and/or concentration information of the sample to be measured according to the oscillation state. Optionally, the activity information and/or concentration information of the sample to be measured may also be displayed through an interaction apparatus.

It should be understood that although each step in the flowchart in FIGS. 3-9 is displayed in succession as indicated by an arrow, these steps are not necessarily executed in succession in the order indicated by the arrows. Unless explicitly described herein, the execution of these steps is not limited to a strict order, instead, the steps may be executed in another order. In addition, at least some steps in FIGS. 3-9 may comprise multiple sub-steps or multiple stages. These sub-steps or stages are not necessarily executed or completed at the same time point, but can be executed at different time points. These sub-steps or stages are also not necessarily executed in succession, but can be executed in turn or alternately with at least some other steps or sub-steps or stages of other steps.

A person of ordinary skill in the art can understand that all or part of the flows in the methods in the above embodiments can be implemented by a computer program by instructing related hardware. The computer program can be stored in a non-volatile computer readable storage medium, and when the computer program is executed, the flows such as the embodiments of the above various methods may be comprised. Any reference to a memory, a database, or other media configured in various embodiments provided in the present application may comprise a non-volatile and/or volatile memory. The non-volatile memory may comprise a read only memory (ROM), a programmable ROM (PROM), an electrically programmable ROM (EPROM), an electrically erasable programmable ROM (EEPROM), or a flash memory. The volatile memory may comprise a random access memory (RAM) or an external cache memory. By way of illustration but not limitation, the RAM is available in a plurality of forms, such as a static RAM (SRAM), a dynamic RAM (DRAM), a synchronous DRAM (SDRAM), a double data rate SDRAM (DDRSDRAM), an enhanced SDRAM (ESDRAM), a Synchlink DRAM (SLDRAM), a Rambus (Rambus) direct RAM (RDRAM), a direct Rambus dynamic RAM (DRDRAM), a Rambus dynamic RAM (RDRAM), and the like.

In an embodiment, provided is computer-readable storage medium(s) with a computer program stored therein, when the computer program is executed by a processor, the steps in the method as described in any one of the above embodiments are implemented. Specifically, when the computer program stored on the storage medium is executed by the processor, the following steps are implemented.

S100, a driving electromagnetic field is generated to drive a metal moving component 110 to oscillate in a sample to be measured in a reaction container.

Specifically, the driving electromagnetic field can be generated by an even number of driving coil groups 210 arranged opposite to each other. For example, the driving electromagnetic field is generated by two driving coil groups 210 arranged opposite to each other, and the driving electromagnetic field can drive the metal moving component 110 to move in the sample to be measured in the reaction container. For example, the metal moving component 110 makes a reciprocating motion or an oscillation in the sample to be measured in the reaction container. Optionally, when a blood coagulation detection is required, the reaction container holding the sample to be measured can be transferred to the sample detection apparatus, then a reagent for the blood coagulation reaction is added into the reaction container, and then the blood coagulation detection starts. Normally, after the blood coagulation reaction starts, the driving electromagnetic field can drive the metal moving component 110 to move in the sample to be measured in the reaction container, and as the blood coagulation reaction progresses, since the fibrin generated will hinder the motion of the metal moving component 110, the amplitude of motion of the metal moving component 110 will gradually decrease.

S200, an inductive electromagnetic field is generated to detect the electrical signal produced by the oscillation of the metal moving component 110 in the driving electromagnetic field.

Specifically, the inductive electromagnetic field can be generated by an even number of measuring coil groups 220 arranged opposite to each other, for example, generated by two measuring coil groups 220 arranged opposite to each other. The inductive electromagnetic field can be configured for detecting the electrical signal produced by the oscillation of the metal moving component 110 in the driving electromagnetic field. Optionally, the measuring coil 220 is connected to the control apparatus, and the measuring coil 220 can transfer the electrical signal of the metal moving component 110 detected thereby to the control apparatus. Optionally, the measuring coil 220 can be connected to the control apparatus through a signal conversion circuit and a signal conditioning circuit. For example, the signal conversion circuit can perform analog-to-digital conversion for the electrical signal of the metal moving component 110 acquired by the measuring coil in at least one continuous time period, and the signal conditioning circuit can perform preprocessing, such as rectification, filtering and normalization, for the electrical signal of the metal moving component 110 acquired by the measuring coil in the at least one continuous time period. FIG. 10 is a schematic diagram of the electrical signal of the metal moving component 110 in a continuous time period in an embodiment.

S300, the electrical signal of the metal moving component 110 in at least one continuous time period is stored.

Specifically, the electrical signal of the metal moving component 110 in the at least one continuous time period can be stored in the storage apparatus. Normally, after a specific reaction reagent is added to the sample to be measured (such as blood) in the reaction container, the blood coagulation reaction starts, and as the blood coagulation reaction progresses, the production of fibrin increases, the viscosity of plasma increases, and the motion intensity of the metal moving component 110 gradually decreases. Of course, when the sample is abnormal or the instrument is abnormal, the situation of motion of the metal moving component 110 may be different accordingly. Therefore, the electrical signal of the metal moving component 110 in the at least one continuous time period can reflect the reaction process of the sample. By means of storing the electrical signal of the metal moving component 110 in the at least one continuous time period, the electrical signal of the metal moving component 110 in the at least one continuous time period can be analysed to determine whether there is an abnormal situation in the detection, thereby improving the reliability and practicability of detection. The at least one continuous time period mentioned here may be preset, or may be dynamically changed according to the actual situation of detection, which is not limited here.

It should be clear that, in the embodiment of the present application, when the processor executes the computer program stored in the storage medium, the execution process thereof is consistent with the execution process of the foregoing method, which can specifically refer to the above description and will not be repeated here.

The technical features of the embodiments described above can be arbitrarily combined, but for brevity, not all possible combinations of the technical features in the embodiments described above have been described. However, as long as there is no contradiction in any combination of these technical features, they should be considered to fall within the scope of disclosure of this specification.

The above embodiments merely represent several implementations of the present application, giving specifics and details thereof, but should not be understood as limiting the scope of the present patent of invention thereby. It should be noted that those of ordinary skill in the art could also make several alterations and improvements without departing from the spirit of the present application and these would all fall within the scope of protection of the present application. Therefore, the scope of protection of the present patent of application shall be in accordance with the appended claims.

## Claims

**1.** A blood coagulation analyser, **characterized in that** the blood coagulation analyser comprises:
a reaction container, and a metal moving component placed in the reaction container, wherein the reaction container is configured to hold a mixed solution of a sample to be measured and a reaction reagent;
a sample detection apparatus, comprising a driving coil and a measuring coil, wherein the driving coil is configured to generate a driving electromagnetic field to drive the metal moving component to oscillate in the sample to be measured in the reaction container; and the measuring coil is configured to generate an inductive electromagnetic field to detect an electrical signal produced by the oscillation of the metal moving component; and
a control apparatus configured for acquiring the electrical signal of the metal moving component in at least one continuous time period;
and a storage apparatus used for storing the electrical signal in the at least one continuous time period.

**2.** The blood coagulation analyser according to claim 1, **characterized in that**, the control apparatus is further used for obtaining oscillation state of the metal moving component according to the electrical signal of the metal moving component in the at least one continuous time period; or the control apparatus is further used for obtaining and outputting the oscillation state of the metal moving component according to the electrical signal of the metal moving component in the at least one continuous time period; and the oscillation state is related to a blood coagulation test result of the sample.

**3.** The blood coagulation analyser according to claim 2, **characterized in that**, the electrical signal of the metal moving component is a voltage signal, and the oscillation state includes a change in signal wave amplitude of the electrical signal.

**4.** The blood coagulation analyser according to claim 2, **characterized in that**, the electrical signal is a voltage signal, the oscillation state includes a first-order derivative of the signal wave amplitude change of the electrical signal and/or a second-order derivative of the signal wave amplitude change of the electrical signal.

**5.** The blood coagulation analyser according to claim 2, **characterized in that**, the blood coagulation analyser further comprises an interaction apparatus connected to the control apparatus, the interaction apparatus being configured for displaying the oscillation state of the metal moving component.

**6.** The blood coagulation analyser according to claim 5, **characterized in that**, the oscillation state of the metal moving component is displayed on the interaction apparatus in the form of a line.

**7.** The blood coagulation analyser according to claim 6, **characterized in that**, the electrical signal of the metal moving component is a voltage signal, and the oscillation state includes a change in signal wave amplitude of the electrical signal; and a horizontal axis of a coordinate system where the line is located is time, and a vertical axis is voltage.

**8.** The blood coagulation analyser according to claim 5, **characterized in that**, the oscillation state of the metal moving component is displayed on the interaction apparatus in the form of a chart or a table.

**9.** The blood coagulation analyser according to claim 2, **characterized in that**, the control apparatus is further configured for obtaining the blood coagulation test result of the sample according to the oscillation state, the blood coagulation test result including clotting time of the sample and/or clotting start time of the sample.

**10.** The blood coagulation analyser according to claim 9, **characterized in that**, the oscillation state includes signal wave amplitude change information of the electrical signal, and the control apparatus is further used for, when the signal wave amplitude of the electrical signal at the current time point is less than or equal to a clotting threshold, obtaining the clotting time according to the current time point.

**11.** The blood coagulation analyser according to claim 10, **characterized in that**, the control apparatus is further used for obtaining the clotting threshold according to the average amplitude value of signal waves before the signal wave at the current time point.

**12.** The blood coagulation analyser according to claim 2, **characterized in that**, the control apparatus is furtherconfigured for determining whether there is a detection abnormality according to the oscillation state of the metal moving component.

**13.** The blood coagulation analyser according to claim 12, **characterized in that**, the control apparatus is further configured for outputting the detection abnormality and/or outputting an operating recommendation and/or giving an alarm according to the oscillation state of the metal moving component.

**4.** The blood coagulation analyser according to claim 2, **characterized in that**, the control apparatus is further configured for outputting clinical reference according to the oscillation state of the metal moving component.

**15.** A sample detection method of a blood coagulation analyser, **characterized in that** the method comprises the following steps:
generating a driving electromagnetic field to drive a metal moving component to oscillate in a sample to be measured in a reaction container;
generating an inductive electromagnetic field to detect an electrical signal produced by the oscillation of the metal moving component in the driving electromagnetic field;
storing the electrical signal of the metal moving component in at least one continuous time period.

**16.** The method according to claim 15, **characterized by** further comprising the following step:
obtaining and outputting an oscillation state of the metal moving component according to the electrical signal of the metal moving component in the at least one continuous time period; or obtaining and outputting motion, the oscillation state of the metal moving component according to the electrical signal of the metal moving component in the at least one continuous time period;

**17.** The method according to claim 16, **characterized in that**, the electrical signal of the metal moving component is a voltage signal, and the oscillation state includes a change in signal wave amplitude of the electrical signal.

**8.** The method according to claim 16, **characterized in that**, the electrical signal is a voltage signal, the oscillation state includes a first-order derivative of the signal wave amplitude change of the electrical signal and/or a second-order derivative of the signal wave amplitude change of the electrical signal.

**19.** The method according to claim 16, **characterized in that**, the electrical signal of the metal moving component is a voltage signal, and the oscillation state includes a change in signal wave vibration frequency of the electrical signal, signal phase of the signal wave or signal wave area.

**20.** The method according to claim 11, **characterized in that** the method further comprises the following step:
outputting the oscillation state of the metal moving component in the form of a line.

**21.** The method according to claim 20, **characterized in that**, the electrical signal of the metal moving component is a voltage signal, and the oscillation state includes a change in signal wave amplitude of the electrical signal; and a horizontal axis of a coordinate system where the line is located is time, and a vertical axis is voltage.

**22.** The method according to claim 16, **characterized by** further comprising:
outputting oscillation state of the metal moving component in the form of a chart or a table.

**23.** The method according to claim 16, **characterized by** further comprising the following step: obtaining a blood coagulation test result of the sample according to the oscillation state, the blood coagulation test result including the clotting time of the sample and/or the clotting start time of the sample.

**24.** The method according to claim 23, **characterized in that**, the oscillation state includes signal wave amplitude change information of the electrical signal, and the method further comprises the following step:
if the signal wave amplitude value of the electrical signal at the current time point is less than or equal to a clotting threshold, obtaining the clotting time according to the current time point.

**25.** The method according to claim 24, **characterized by** further comprising the following step: obtaining the clotting threshold according to the average amplitude value of signal waves before the signal wave at the current time point.

**26.** The method according to claim 24, **characterized by** further comprising:
obtaining an initial value of the clotting threshold according to the average value of signal wave amplitude values of the first N signal waves in the electrical signal of the metal moving component, wherein N is greater than or equal to 1, and N is a positive integer.

**27.** The method according to claim 16, **characterized by** further comprising the following step:
obtaining and/or outputting the clotting start time according to the oscillation state.

**28.** The method according to claim 16, **characterized by** further comprising the following step:
determining whether there is a detection abnormality according to the oscillation state.

**29.** The method according to claim 28, **characterized in that**, the oscillation state includes signal wave amplitude change information of the electrical signal, and the method further comprises the following step:
if all the signal wave amplitude values of the first M signal waves of the electrical signal are less than the preset minimum amplitude threshold, it is determined that there is a detection abnormality;
wherein M is greater than or equal to 1, and M is a positive integer.

**30.** The method according to claim 28, **characterized in that**, the oscillation state includes signal wave amplitude change information of the electrical signal, and the method further comprises the following step:
if the signal wave amplitude value of the electrical signal starts to decay in the preset maximum reaction time period, and the signal wave amplitude value of the electrical signal in the preset maximum reaction time period remains greater than the clotting threshold, determining that there is a detection abnormality.

**31.** The method according to claim 28, **characterized in that**, the oscillation state includes signal wave amplitude change information of the electrical signal, and the method further comprises the following step:
if there is a sudden change in the signal wave amplitude value of the electrical signal, determining that there is a detection abnormality.

**32.** The method according to claim 28, **characterized by** further comprising the following step: outputting abnormal information and/or outputting operating recommendation information and/or giving alarm according to the oscillation state.

**33.** The method according to claim 16, **characterized by** further comprising the following step: outputting activity information and/or concentration information of the sample to be measured according to the oscillation state.

**34.** The method according to claim 16, **characterized by** further comprising the following step: outputting clinical reference information according to the oscillation state.

**35.** Computer-readable storage medium(s) with a computer program stored therein, wherein when the computer program is executed by a processor, the steps in the method of any one of claims 15-34 are implemented.
